# EUROPEAN PATENT APPLICATION

(11) **EP 4 643 764 A1**
(43) Date of publication of application: **05.11.2025**
(21) Application number: 24172932.6
(22) Date of filing: 29.04.2024
(51) Int. Cl.: A61B 5/01, A61B 5/00, A61F 13/00

(54) **TEMPERATURE SENSING ARRANGEMENT, AND ABSORBENT ARTICLE COMPRISING TEMPERATURE SENSING ARRANGEMENT**

(71) Applicant: Mölnlycke Health Care AB, 431 21 Mölndal (SE)
(72) Inventor: WIRTHS, Walter, 82024 TAUFKIRCHEN (DE); MARTAENG, Rasmus, 412 81 GÖTEBORG (SE)
(74) Representative: Kransell & Wennborg KB

(57) **Abstract**

A temperature sensing arrangement (5) for inclusion in an absorbent article (1) for sensing a temperature distribution across an area of a body of a user using the absorbent article (1). The temperature sensing arrangement (5) comprises a fluid-permeable carrier (23) including a plurality of conductors (25a-h); and a plurality of temperature sensing elements (27) distributed across the carrier (23), each temperature sensing element (27) of the plurality of temperature sensing elements being electrically connected to a unique combination of two conductors of the plurality of conductors (25a-h).

## Description

### Field of the Invention

The present invention relates to a temperature sensing arrangement, and an absorbent article comprising such a temperature sensing arrangement.

### Background of the Invention

The temperature distribution across an area of the body of a user may be an interesting indicator of a present state of that area. For instance, the temperature distribution may provide information about the present condition of a wound covered by a wound dressing.

It would therefore be desirable to provide for measurement of the temperature distribution across an area of the body of a user.

### Summary

It is an object of the present invention to provide for measurement of the temperature distribution across an area of the body of a user.

According to an aspect of the present invention, it is therefore provided a temperature sensing arrangement for inclusion in an absorbent article for sensing a temperature distribution across an area of a body of a user using the absorbent article, the temperature sensing arrangement comprising: a fluid-permeable carrier including a plurality of conductors; and a plurality of temperature sensing elements distributed across the carrier, each temperature sensing element of the plurality of temperature sensing elements being electrically connected to a unique combination of two conductors of the plurality of conductors.

The function of the carrier is to support the temperature sensing elements in such a way that the temperature sensing elements are spatially distributed as desired, while being electrically connected to respective electrical conductors. Accordingly, the carrier may be provided by the plurality of conductors themselves, or may include a substrate on or in which the conductors are provided.

The present invention is based on the realization that measurement of a temperature distribution across an area of the body of a user can be combined with the desired functionality of liquid absorption by an absorbent article covering the same area, by providing the temperature sensing arrangement with a fluid-permeable carrier including a plurality of conductors, and a plurality of temperature sensing elements distributed across the carrier, each temperature sensing element of the plurality of temperature sensing elements being electrically connected to a unique combination of two conductors of the plurality of conductors.

It should be understood that the term "fluid" is not limited to mean "liquid", but also covers, for example, vapor. Accordingly, in example configurations of the temperature sensing arrangement, the fluid-permeable carrier may allow passage therethrough of liquid (and vapor), and in other example configurations, the fluid-permeable carrier may allow passage of vapor, but not of liquid.

Through the fluid-permeability of the temperature sensing arrangement according to examples of the present invention, the temperature distribution across the area of the body of the user can be measured while still providing for fluid transport from the area of the body of the user into and out of an absorbent article.

The temperature sensing arrangement according to examples of the present invention may also be used separately, without being included in an absorbent article. In such applications, the fluid-permeability of the carrier of the temperature sensing arrangement may reduce the risk of discomfort to the user and/or skin rash, and/or harmful maceration of periwound skin due to accumulation of wound fluid, etc..

According to an example configuration, the temperature sensing arrangement may further comprise readout circuitry coupled to each conductor of the plurality of conductors, and configured to address each temperature sensing element of the plurality of temperature sensing elements. The readout circuitry may be configured to address the individual temperature sensing elements sequentially, or several temperature sensing elements in parallel.

In another configuration, the temperature sensing arrangement and the readout circuitry may be provided as separate devices. For instance, the temperature sensing arrangement may comprise a readout interface for electrical connection with readout circuitry. In example configurations where the temperature sensing arrangement is included in an absorbent article, such a readout interface may be accessible from the outside of the absorbent article, when the absorbent article is arranged on the user.

According to examples of the temperature sensing arrangement of the present invention, for each temperature sensing element of the plurality of temperature sensing elements, the two conductors electrically connected to the temperature sensing element may be crossing each other, without direct conductive contact between the two conductors.

According to an example configuration of the temperature sensing arrangement, a first set of conductors included in the plurality of conductors may extend in parallel with each other in a first direction; and a second set of conductors included in the plurality of conductors may extend in parallel with each other in a second direction.

In example configurations, the first set may comprise conductors extending in the first direction as well as in the second direction, and the second set may comprise conductors extending in the second direction as well as in the first direction. In other words, one or several conductors may extend in the first direction along a first portion, and extend in the second direction along a second portion. Compared to row-column multiplexing where each temperature sensing element is connected to a row conductor and to a column conductor, this conductor configuration may provide for the use of so-called high density analog multiplexing, which requires fewer readout conductors for addressing a given number of temperature sensing elements. This may, in turn, provide for improved fluid-permeability of the temperature sensing arrangement, for a given type of temperature sensing elements and spacing between temperature sensing elements.

According to an example of the temperature sensing arrangement of the present invention, at least one half of an area defined by two mutually adjacent conductors extending in the first direction and two mutually adjacent conductors extending in the second direction may be open to passage of fluid. It should be understood that the passage of fluid may take place through the temperature sensing arrangement, from one side of the temperature sensing arrangement to the opposite side of the temperature sensing arrangement. When the temperature sensing arrangement is in use, fluid, such as liquid and/or vapor, may, for example, pass from the side of the temperature sensing arrangement facing the body of the user to the opposite side of the temperature sensing arrangement, facing away from the body of the user. Depending on the configuration of the temperature sensing arrangement, the portion thereof that is open to passage of fluid may be provided as relatively few relatively large openings, and/or as relatively many relatively small openings, such as perforations.

According to examples, at least one half of a total area of the temperature sensing arrangement may be open to passage of fluid.

According to an example configuration, the temperature sensing arrangement may be substantially rectangular. At least a portion of the temperature sensing arrangement where conductors and/or temperature sensing elements are arranged may be substantially rectangular, so that the temperature sensing arrangement can be said to have a first diagonal and a second diagonal crossing the first diagonal.

According to an example configuration, each conductor in the plurality of conductors may extend in a direction substantially in parallel with a diagonal of the temperature sensing arrangement. This may provide for high density analog multiplexing addressing of temperature sensing elements that may be substantially evenly distributed across a sensing surface portion of the temperature sensing arrangement.

According to an example configuration, each conductor in the plurality of conductors may be connected to the same number of temperature sensing elements. For example, this number may be equal to the total number of conductors minus one, which would provide for the highest ratio between the number of temperature sensing elements and conductors.

According to an example configuration, each conductor in a subset of the plurality of conductors may extend partly substantially in parallel with a first diagonal of the temperature sensing arrangement and partly substantially in parallel with a second diagonal of the temperature sensing arrangement. This configuration may provide for a particularly high ratio between the number of temperature sensing elements and the number of conductors comprised in the temperature sensing element. This may, in turn, provide for a relatively high fluid-permeability, especially liquid-permeability, of the temperature sensing arrangement.

According to example configurations, the fluid-permeable carrier may comprise a flexible substrate; and the plurality of conductors may be provided on the flexible substrate. These configurations allow for rational manufacturing of the temperature sensing arrangement using well-established manufacturing techniques. The carrier may, for example, be produced as a flexible printed circuit board, or as printed electronics, where the conductors may be printed on a carrier. As an alternative, the carrier may be provided in the form of a textile with integrated conductors.

According to an example configuration, each temperature sensing element may be a thermistor. This configuration may provide for a cost-efficient solution. The thermistors may be provided as surface mounted components, or may be provided in the form of one or more layers of printed thermistor material.

According to examples, the temperature sensing arrangement may comprise at least 28 temperature sensing elements.

The temperature sensing arrangement according to various example configurations of the present invention may be included in an absorbent article, further comprising a fluid absorbing pad configured to absorb fluid from a body of a user.

According to an example, the absorbent article may comprise a body contacting layer and a backing layer sandwiching the fluid absorbing pad; and the temperature sensing arrangement may be arranged between the body contacting layer and the backing layer.

According to an example, the temperature sensing arrangement may be arranged between the body contacting layer and the fluid absorbing pad, which may provide for improved heat conduction between each temperature sensing element and the body of the user. Alternatively, the temperature sensing arrangement may be sandwiched between layers of the fluid absorbing pad. Another configuration could be that the temperature sensing arrangement according to an example may be arranged to be in direct contact with the body, when the absorbent article is attached to the body. In such an example configuration of the absorbent article, the body contacting layer may be in contact with the body through openings in the temperature sensing arrangement. This example configuration would provide improved heat conduction between the body and the temperature sensing elements. Alternatively, the body contacting layer may be provided with openings accommodating the temperature sensing elements.

### Brief Description of the Drawings

These and other aspects of the present invention will now be described in more detail, with reference to the appended drawings showing example embodiments of the invention, wherein:
Fig 1 is an illustration of an exemplary absorbent article, in the form of a wound dressing, arranged on the arm of a user;
Fig 2 is an exploded view of the absorbent article in fig 1, with an embedded temperature sensing arrangement according to an example embodiment of the present invention; and
Fig 3 is a top-view of a temperature sensing arrangement according to an example of the invention.

### Detailed Description of Example Embodiments

The present invention will now be described more fully hereinafter with reference to the accompanying drawings, in which example embodiments of the present invention are shown. The present invention may, however, be embodied in many different forms and should not be construed as limited to the embodiments set forth herein; rather, these embodiments are provided for thoroughness and completeness, and fully convey the scope of the present invention to the skilled person. Like reference characters refer to like elements throughout.

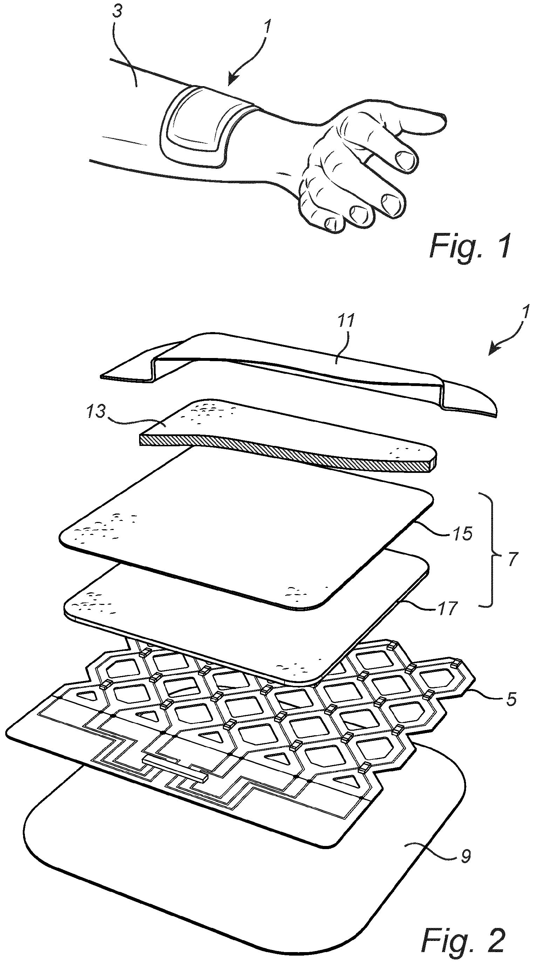

Fig 1 is an illustration of an exemplary absorbent article 1, in the form of a wound dressing, arranged on the arm 3 of a user. A wound dressing, such as that schematically shown in fig 1 may be a particularly interesting application for the temperature sensing arrangement according to embodiments of the present invention, since the temperature distribution across the wound area may provide an indication of the state of the wound. For instance, the temperature distribution may provide a qualitative and quantitative indication of an ongoing infection, without the need to remove the wound dressing.

Fig 2 is an exploded view of the absorbent article 1 in fig 1, including an embedded temperature sensing arrangement 5 according to an example embodiment of the present invention. The absorbent article (wound dressing) 1 in fig 2 comprises a fluid absorbing pad 7 configured to absorb fluid from the body of the user. In this particular case, the fluid may be exudate from a wound covered by the wound dressing. For other kinds of absorbent articles, the fluid may be urine or blood.

In this example configuration, the absorbent article 1 comprises a body contacting layer 9 and a backing layer 11 sandwiching the fluid absorbing pad 7 and the temperature sensing arrangement 5. The fluid absorbing pad 7 is, in this example configuration, a layered structure comprising a fluid retention layer 13, a fluid distribution layer 15, and a fluid absorption layer 17. In the fluid absorbing pad 7. The fluid absorption layer 17 is arranged closest to the body contacting layer 9, the fluid distribution layer 15 is arranged on the fluid absorption layer 17, and the fluid retention layer 13 is arranged on the fluid distribution layer 15. The temperature sensing arrangement 5 may advantageously be arranged between the body contacting layer 9 and the fluid absorption layer 17. In embodiments, however, the temperature sensing arrangement 5 may be arranged inside the fluid absorbing pad 7, such as between the fluid retention layer 13 and the fluid distribution layer 15. In other embodiments, the temperature sensing arrangement 5 may be arranged between the fluid absorbing pad and the backing layer 11, or between the body contacting layer 9 and the body of the user.

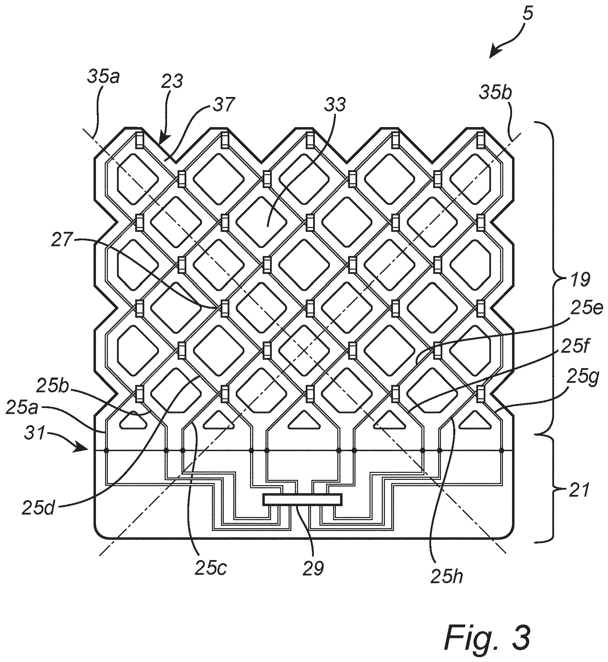

The temperature sensing arrangement 5 according to an example of the present invention will now be described with reference to fig 3. In the example configuration of fig 3, the temperature sensing arrangement 5 comprises a sensing portion 19, and a readout portion 21. The sensing portion 19 comprises a fluid-permeable carrier 23 including a plurality of conductors 25a-h, and a plurality of temperature sensing elements 27 distributed across the carrier 23 (only one of the temperature sensing elements is indicated by a reference numeral to avoid cluttering the drawing). The readout portion 21 comprises readout circuitry 29.

As can be seen in fig 3, each temperature sensing element is electrically connected to a unique combination of two conductors of the plurality of conductors 25a-h. For example, the temperature sensing element 27 indicated by the reference numeral in fig 3 is the only temperature sensing element 27 that is electrically connected to the conductors 25a and 25f. This means that the temperature sensing element 27 can be uniquely addressed using the conductors 25a and 25f. In example configurations where the temperature sensing element 27 is a thermistor, which may for example be provided as a surface mounted component or a printed component, a sensing value indicative of the temperature of the temperature sensing element 27 can be acquired by detecting the resistance of the thermistor. This may, for example, be done by measuring the current through the thermistor when a known voltage is applied across the thermistor, or by measuring the voltage across the thermistor when a known current is passed through the thermistor. Each temperature sensing element 27 may be covered by an electrically insulating material (not shown in fig 3).

In the example configuration in fig 3, each temperature sensing element 27 can be addressed and sampled by the readout circuitry via a readout interface 31 between the sensing portion 19 and the readout portion 21. In this example configuration, where the readout portion 21 is included in the temperature sensing arrangement 5, sensing values indicative of the temperature of each temperature sensing element 27 may be acquired repeatedly and stored locally and/or transmitted, providing for an evaluation of a development over time of a temperature distribution across an area of the body of the user. Alternatively, only the part in fig 3 indicated as the sensing portion 19 may be included in the temperature sensing arrangement 5, and readout can be carried out by contacting the readout interface 31 by a separate readout circuit. In that case, the readout interface 31 may be an externally accessible connector.

In the example configuration of fig 3, the fluid-permeability of the carrier 23 is provided by large openings 33 (only one of these is indicated by a reference numeral to avoid cluttering the drawing) allowing passage of fluid through the temperature sensing arrangement 5. Each opening 33 may have an area being at least one half of an area defined by mutually adjacent conductors of the carrier 23. For the indicated opening 33, the mutually adjacent conductors are 25a, 25b, 25g, and 25h. In the example configuration of fig 3, the openings 33 are exemplified as being single large area openings. As will be immediately apparent to one of ordinary skill in the relevant art, the fluid-permeability of the carrier 23 may alternatively be achieved by a larger number of smaller openings, such as a large number of perforations. In configurations where the carrier is provided in the form of a textile, the openings may be defined by spacings between yarns.

In the example configuration of fig 3, the sensing portion 19 of the temperature sensing arrangement 5 is substantially rectangular, and each conductor 25a-h in the plurality of conductors extends in a direction substantially in parallel with a diagonal of the sensing portion 19. As was pointed out above, in example configurations, the sensing portion 19 may constitute the temperature sensing arrangement 5. Furthermore, each conductor 25b-g in a subset of the conductors extends partly substantially in parallel with a first diagonal 35a and partly substantially in parallel with a second diagonal 35b. This configuration allows the use of so-called high density analog multiplexing (HDAM) (also sometimes referred to as charlieplexing) for readout of sensing values from substantially evenly distributed temperature sensing elements 27. This configuration allows the use of fewer conductors for acquiring sensing values from a given number of temperature sensing elements 27, which in turn provides for improved fluid-permeability for a given sensing resolution. In the case of the readout circuitry 29 being included in a separate and reusable device, this configuration may also reduce the number of contacts, for a given number of temperature sensing elements. This provides for a less complex and more compact readout device, which may be cost-efficient.

As is schematically indicated in fig 3, the fluid-permeable carrier 23 may comprise a flexible substrate 37, and the conductors 25a-h may be provided on the flexible substrate 37. As will be apparent to one of ordinary skill in the relevant art, there are many different types of flexible substrates that can be used. According to one example, conventional flexible printed circuit board technology may be used, where the conductors are provided as one or more etched conductor patterns on a polyimide substrate. According to another example, techniques for printed electronics may be used, where the conductors are printed on a substrate using conductive ink. In the latter case, insulation ink may be used to prevent short circuits at conductor crossings.

As an alternative to the example configuration illustrated in fig 3, the conductors may be arranged in rows and columns, and each temperature sensing element may be connected to a unique combination of a row conductor and a column conductor.

In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage.

## Claims

1. A temperature sensing arrangement (5) for inclusion in an absorbent article (1) for sensing a temperature distribution across an area of a body of a user using the absorbent article (1), the temperature sensing arrangement (5) comprising:
a fluid-permeable carrier (23) including a plurality of conductors (25a-h); and
a plurality of temperature sensing elements (27) distributed across the carrier (23), each temperature sensing element (27) of the plurality of temperature sensing elements being electrically connected to a unique combination of two conductors of the plurality of conductors (25a-h).

2. The temperature sensing arrangement (5) according to claim 1, further comprising readout circuitry (29) coupled to each conductor of the plurality of conductors (25a-h), and configured to address each temperature sensing element (27) of the plurality of temperature sensing elements.

3. The temperature sensing arrangement (5) according to claim 1 or 2, wherein, for each temperature sensing element (27) of the plurality of temperature sensing elements, the two conductors electrically connected to the temperature sensing element are crossing each other.

4. The temperature sensing arrangement (5) according to any one of the preceding claims, wherein:
a first set of conductors included in the plurality of conductors (25a-h) extend in parallel with each other in a first direction; and
a second set of conductors included in the plurality of conductors (25a-h) extend in parallel with each other in a second direction.

5. The temperature sensing arrangement (5) according to claim 4, wherein at least one half of an area defined by two mutually adjacent conductors extending in the first direction and two mutually adjacent conductors extending in the second direction is open to passage of fluid.

6. The temperature sensing arrangement (5) according to any one of the preceding claims, wherein at least one half of a total area of the temperature sensing arrangement (5) is open to passage of fluid.

7. The temperature sensing arrangement (5) according to any one of the preceding claims, wherein the temperature sensing arrangement (5) is substantially rectangular.

8. The temperature sensing arrangement (5) according to claim 7, wherein each conductor in the plurality of conductors (25a-h) extends in a direction substantially in parallel with a diagonal (35a; 35b) of the temperature sensing arrangement (5).

9. The temperature sensing arrangement (5) according to claim 8, wherein each conductor in the plurality of conductors (25a-h) is connected to the same number of temperature sensing elements (27).

10. The temperature sensing arrangement (5) according to any one of claims 7 to 9, wherein each conductor in a subset of the plurality of conductors (25a-h) extends partly substantially in parallel with a first diagonal (35a) of the temperature sensing arrangement (5) and partly substantially in parallel with a second diagonal (35b) of the temperature sensing arrangement (5).

11. The temperature sensing arrangement (5) according to any one of the preceding claims, wherein the fluid-permeable carrier (23) comprises:
a flexible substrate (37); and
the plurality of conductors (25a-h) are provided on the flexible substrate (37).

12. The temperature sensing arrangement (5) according to any one of the preceding claims, wherein each temperature sensing element (27) is a thermistor.

13. The temperature sensing arrangement (5) according to any one of the preceding claims, comprising at least 28 temperature sensing elements (27).

14. An absorbent article (1) comprising:
a fluid absorbing pad (7) configured to absorb fluid from a body of a user; and
the temperature sensing arrangement (5) according to any one of the preceding claims.

15. The absorbent article (1) according to claim 14, wherein:
the absorbent article (1) comprises a body contacting layer (9) and a backing layer (11) sandwiching the fluid absorbing pad (7); and
the temperature sensing arrangement (5) is arranged between the body contacting layer (9) and the backing layer (11).
